# EUROPÄISCHE PATENTSCHRIFT

(11) **EP 2 054 020 B1**
(45) Veröffentlichungstag und Bekanntmachung des Hinweises auf die Patenterteilung: **02.10.2013**
(21) Anmeldenummer: 07788306.4
(22) Anmeldetag: 08.08.2007
(51) Int. Cl.: A61K 8/46, A61K 8/49, A61Q 7/00, A61P 17/14, A61Q 5/06

(54) **KOSMETISCHES MITTEL ENTHALTEND PURIN UND/ODER EIN PURINDERIVAT UND TAURIN**
COSMETIC COMPOSITION COMPRISING PURINE AND/OR A PURINE DERIVATIVE AND TAURINE
AGENT COSMÉTIQUE CONTENANT DE LA PURINE ET/OU UN DÉRIVÉ DE PURINE ET DE LA TAURINE

(30) Priorität: 20.10.2006 DE 102006050398
(43) Veröffentlichungstag der Anmeldung: 06.05.2009
(73) Patentinhaber: Henkel AG & Co. KGaA, 40589 Düsseldorf (DE)
(72) Erfinder: KNAPPE, Thorsten, 22869 Schenefeld (DE); WALTER, Helen, 22301 Hamburg (DE); WEISS, Thomas, 40591 Düsseldorf (DE); SCHULZE ZUR WIESCHE, Erik, 20144 Hamburg (DE); GIESEN, Melanie, 47608 Geldern (DE); SPUHLER, Philipp, 40477 Düsseldorf (DE)
(86) Internationale Anmeldenummer: PCT/EP2007/058217
(87) Internationale Veröffentlichungsnummer: WO 2008/046676

(56) Entgegenhaltungen:
- EP-A- 1 685 826
- WO-A-02/074265
- WO-A-2007/003307
- US-A1- 2005 175 565
- SEIDL R ET AL: "A taurine and caffeine-containing drink stimulates cognitive performance and well-being" AMINO ACIDS, SPRINGER VERLAG, AU, Bd. 19, Nr. 3-4, 2000, Seiten 635-642, XP002385036 ISSN: 0939-4451

## Beschreibung

Die vorliegende Erfindung betrifft kosmetische Mittel enthaltend Coffein und Taurin und die Verwendung dieser Mittel.

Haarwachstum ist kein kontinuierlicher Vorgang, sondern verläuft in Wachstums- und Haarverlustschüben, die sich cyclisch wiederholen. Dieser Vorgang ist im Wesentlichen darauf zurückzuführen, dass Haarfollikelzellen einem genetisch festgelegten Zyklus von Wachstum, Regression und Ruhephase unterliegen. Der Haarfollikel ist damit das einzige Organ, das sich ständig selbst erneuert und somit einen, in Abhängigkeit von der jeweiligen Wachstumsphase, einzigartigen Metabolismus aufweist. So kommt der Metabolismus des Haarfollikels in der Ruhephase fast völlig zum Erliegen und wird mit jedem neuen Beginn eines weiteren Zyklus ebenfalls neu initiiert. Gesteuert wird dieser Zyklus von einer kleinen, hochspezialisierten Zellpopulation im Haarbulbus, den dermalen Papillenzellen, die durch ein einzigartiges komplexes System molekularer Signale, die spezifisch für jede Phase des Haarzyklus sind, das Haarwachstum kontrolliert. Im Laufe des cyclischen Vorgangs kommt es kontinuierlich zum Ausfall einzelner Haare, wobei ein Haarausfall von bis zu 100 Haaren pro Tag als physiologisch normal bezeichnet wird. Ist der komplexe Metabolismus des Haarfollikels gestört, kann es zum vorzeitigen oder übermäßigen Ausfall der Haare und schließlich zu Alopezie bis hin zu vollständiger Glatzenbildung kommen. Die Ursachen von vorzeitigem oder übermäßigem Haarausfall sind vielfältig und reichen von genetischer Veranlagung, Mängelzuständen, Vergiftungen bis hin zu Infektionen und Haarausfall im Zuge einer Chemotherapie.

Es wurde bereits eine Vielzahl von kosmetischen Mitteln zur Verminderung von vorzeitigem Haarausfall und/oder zur Förderung des Haarwachstums entwickelt, die unterschiedlichste Wirkstoffe enthalten.

Da es zur Verminderung von vorzeitigem Haarausfall und/oder zur Förderung des Haarwachstums wesentlich darauf ankommt, den Metabolismus des Haarfollikels positiv zu beeinflussen, muss gewährleistet werden, dass die Wirkstoffe mit den Haarfollikeln wechselwirken können. Dabei hat es sich als zweckmäßig erwiesen, entsprechende kosmetische Mittel in einer Form zu konfektionieren, die bei bestimmungsgemäßer Anwendung mit der Kopfhaut in Kontakt kommt. Typischerweise handelt es sich um Haarwässer, Shampoos oder Haarspülungen und -kuren.

So offenbart etwa EP 1 685 826 A1 die Verwendung kosmetischer Mittel, die anionische und amphotere oberflächenaktive Stoffe (Tenside), die Haarwurzeln stabilisierende Stoffe, insbesondere Minoxidil oder Finasterid, und Coffein enthalten, zur Behandlung erblich bedingten Haarausfalls. Bei den Mitteln gemäß den Beispielen handelt es sich um Shampoos, Haarspülungen oder Haar- und Kopfhautkuren. Die Zugabe von Coffein stabilisiert dabei die Wachstumsvoraussetzungen für den Haarfollikel.

Der Einsatz von Xanthin und dessen Derivate, insbesondere Coffein, zur Behandlung von Haarausfall ist auch aus anderen Schriften hinreichend bekannt. In WO 2004/004672 A1 wird deren Einsatz in Kombination mit einer α-Hydroxysäure, vorzugsweise Zitronensäure beschrieben.

Dennoch besteht nach wie vor Bedarf an entsprechenden Mitteln mit erhöhter Wirksamkeit, die insbesondere vorbeugend eingesetzt werden können.

Aufgabe der vorliegenden Erfindung war es daher, kosmetische Mittel zur Verfügung zu stellen, die besonders wirksam vorzeitigen Haarausfall vermindern und/oder das Haarwachstum fördern. Dabei sollten die Mittel nach Möglichkeit eine langanhaltende Wechselwirkung von Wirkstoff und Haarfollikeln erlauben.

Es wurde nunmehr überraschenderweise gefunden, dass dies durch kosmetische Mittel erreicht werden kann, die eine Kombination von Coffein und Taurin enthalten.

Ein erster Gegenstand der vorliegenden Erfindung ist daher ein kosmetisches Mittel, enthaltend in einem kosmetisch akzeptablen Träger, jeweils bezogen aus das gesamte Mittel
a) 0,001 bis 0,25 Gew.-% Coffein und
b) 0.01 bis 1 Gew.-% 2-Aminoethansulfonsäure.

Untersuchungen an dermalen Papillenzellen haben überraschenderweise gezeigt, dass die beiden Komponenten a) und b) in synergistischer Weise zusammenwirken und im Vergleich zum Einsatz der Einzelkomponenten eine unerwartete Erhöhung der Wirksamkeit erzielt wird. So wurde festgestellt, dass der Einsatz der erfindungsgemäßen Mittel zu einer Anregung des Haarwachstums und einer Verminderung vorzeitigen Haarausfalls führen. Es konnte beispielsweise die synergistische Ausschüttung des Wachstumsfaktors HGF (Hepatozytenwachstumsfaktor, Hepatocyte Growth Factor) festgestellt werden. Ebenfalls wird die Aktivität des Gens für IGFBP3 (Insulin-like Growth Factor Binding Protein 3) in synergistischer Weise verringert. Zusätzlich konnte die signifikante Erhöhung der Aktivität des Gens für HGF nachgewiesen werden. Ferner führte die Behandlung der dermalen Papillenzellen mit Kombinationen eines Purinderivats mit Taurin zur tendenziellen Aktivierung des Gens für KGF (Keratinozytenwachstumsfaktor, Keratinocyte Growth Factor) in dermalen Papillenzellen (siehe Beispiele B und C).

Als ersten zwingenden Bestandteil enthalten die erfindungsgemäßen kosmetischen Mittel Coffein.

Als zweiten zwingenden Bestandteil enthalten die erfindungsgemäßen Mittel bezogen auf das gesamte Mittel 0.01 bis 1 Gew.-% 2-Aminoethansulfonsäure.

2-Aminoethansulfonsäure ist auch unter dem Trivialnamen Taurin bekannt und ist in der Natur weit verbreitet. Es kommt im gesamten Tierreich ubiquitär vor und ist auch in den Samen bestimmter Pflanzen enthalten. Taurin kann aus diesen natürlichen Quellen isoliert werden, wird industriell jedoch aus den Rohstoffen Ethen, Ammoniak und Natriumsulfit gewonnen. Es kommt als Zwischenprodukt bei der Herstellung von Farbstoffen, Arzneimitteln und Reinigungsmitteln zum Einsatz und wird seit einiger Zeit auch als angeblich leistungssteigernder Zusatz Energy Drinks und Sportlernahrung zugegeben.

Die erfindungsgemäßen Mittel enthalten 2-Aminoethansulfonsäure bezogen auf das gesamte Mittel vorzugsweise in Mengen von 0,01 bis 1 Gew.-%.

Die erfindungsgemäßen Mittel enthalten Coffein und 2-Aminoethansulfonsäure in einem kosmetisch akzeptablen Träger.

Bevorzugte kosmetisch akzeptable Träger sind wässrige, alkoholische oder wässrigalkoholische Medien. Als alkoholische Komponente kommen dabei niedere Alkanole sowie Polyole wie Propylenglykol und Glycerin zum Einsatz. Ethanol und Isopropanol sind bevorzugte Alkohole. Wasser und Alkohol können in der wässrig alkoholischen Basis in einem Gewichtsverhältnis von 1:10 bis 10:1 vorliegen. Wasser sowie wässrig-alkoholische Mischungen, die bis zu 50 Gew.-%, insbesondere bis zu 25 Gew.-%, Alkohol, bezogen auf das Gemisch Alkohol/Wasser, enthalten, können erfindungsgemäß bevorzugte Grundlagen sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Der pH-Wert dieser Zubereitungen kann prinzipiell bei Werten von 2 - 11 liegen. Er liegt bevorzugt zwischen 2 und 7. Die Angaben zum pH-Wert beziehen sich dabei im Sinne dieser Schrift auf den pH-Wert bei 25°C, sofern nichts anderes vermerkt ist. Zur Einstellung dieses pH-Wertes kann praktisch jede für kosmetische Zwecke verwendbare Säure oder Base verwendet werden. Üblicherweise werden als Säuren Genusssäuren verwendet. Unter Genusssäuren werden solche Säuren verstanden, die im Rahmen der üblichen Nahrungsaufnahme aufgenommen werden und positive Auswirkungen auf den menschlichen Organismus haben. Genusssäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt. Bevorzugte Basen sind Ammoniak, Alkalihydroxide, Triethanolamin sowie N,N,N',N'-Tetrakis-(2-hydroxypropyl)-ethylendiamin.

In einer besonderen Ausführungsform enthalten die erfindungsgemäßen Mittel neben Purin und/oder Purinderivat(en) und 2-Aminoethansulfonsäure mindestens einen weiteren Wirkstoff, der Haarausfall verhindert und/oder das Haarwachstum fördert. Geeignet sind alle bekannten haarwuchsstimuliernden Wirkstoffe, beispielsweise Minoxidil (6-Piperidino-2,4-pyrimidindiamin-3-oxid), Aminexil (Diaminopyrimidinoxid), Finasterid (N-tert-Butyl-3-oxo-4-aza-5α-androst-1-en-17β-carboxamid), Carnitintartrat und 5-α-Reduktaseinhibitoren.

Als 5-α-Reduktaseinhibitoren sind insbesondere geeignet die funktionellen C₂-C₁₂-Carbonsäuren und deren physiologisch verträglichen Metallsalze, insbesondere 10-Hydroxydecansäure, 10-Hydroxydecensäure und ihren Derivate, Derivate von C₃-C₉-Polyolen, Phenolderivate, Pflanzenextrakte, Riechstoffe, Flavonoide, Isoflavonoide, 6,7-disubstituierte 2,2-Dialkylchromane oder -chromene, Aluminiumchlorohydrat, 2-Phenylethanol, Etidronsäure, 7-Acetyl-1,1,3,4,4,6-hexamethyltetralin, Tropolonderivate, Ester der Schwefelsäure mit alkoxylierten C₈-C₁₈-Fettalkoholen und deren physiologisch verträgliche Metallsalze, Ester der Phosphorsäure und der Triphosphorsäure mit ein- bis sechswertigen Hydroxyverbindungen, Kieselsäureester, aus marinen Organismen isolierbare mycosporin-ähnliche Aminosäuren (MAA) sowie quaternäre Siliconverbindungen. Unter Derivaten sind dabei insbesondere deren Salze, Ester und Amide zu verstehen.

Die erfindungsgemäßen Mittel können weiterhin die Hilfs- und Zusatzstoffe enthalten, die üblicherweise den jeweiligen kosmetischen Mitteln zugesetzt werden.

Demgemäß können die erfindungsgemäßen Mittel beispielsweise zusätzliche Bestandteile enthalten, ausgewählt aus der Gruppe, umfassend Proteinhydrolysate, filmbildende Substanzen, weitere haarwuchsstimulierende Wirkstoffe, nichtionogene Tenside, anionische Tenside, kationische Tenside, zwitterionische Tenside, ampholytische Tenside, nichtionische Polymere, Verdickungsmittel, Parfümöle, Farbstoffe, Lichtschutzmittel, Antioxidantien, Antischuppenwirkstoffe, Treibmittel, Reduktionsmittel.

Als geeignete Hilfs- und Zusatzstoffe sind insbesondere Pflegestoffe zu nennen. Diese können bei geeigneter Wahl des Pflegestoffs beispielsweise in Shampoos, Haarspülungen, Haarkuren, Haarwasser, Haarcreme, Haarlotionen, Haartinkturen, Gele, Pump- und Aerosolsprays und Schaumprodukte eingearbeitet werden.

Als Pflegestoff kann beispielsweise ein Silikonöl und/oder ein Silikongum eingesetzt werden. In einer besonderen Ausführungsform der Erfindung enthalten die Mittel mindestens ein Silikonöl und/oder ein Silikongum.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole (S1) verstanden. Diese können sowohl linear als auch verzweigt als auch cyclisch oder cyclisch und verzweigt sein. Lineare Dimethiconole können durch die folgende Strukturformel (S1 - 1) dargestellt werden:

(HOSiR¹₂) - O - (SiR²₂ - O-)ₓ-(SiR¹₂OH) (S1 - I)

Verzweigte Dimethiconole können durch die Strukturformel (S1 - II) dargestellt werden:

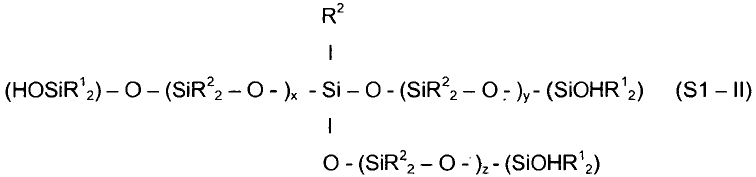

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, insbesondere bevorzugt ist R¹ und R² Methyl. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone (S2) bilden die zweite Gruppe der Silikone, welche erfindungsgemäß enthalten sein können. Diese können sowohl linear als auch verzweigt als auch cyclisch oder cyclisch und verzweigt sein. Lineare Dimethicone können durch die folgende Strukturformel (S2 - I) dargestellt werden:

(SiR¹₃)-O-(SiR¹R²-O-)ₓ-(SiR¹₃) (S2 - I)

Verzweigte Dimethicone können durch die Strukturformel (S2 - II) dargestellt werden:

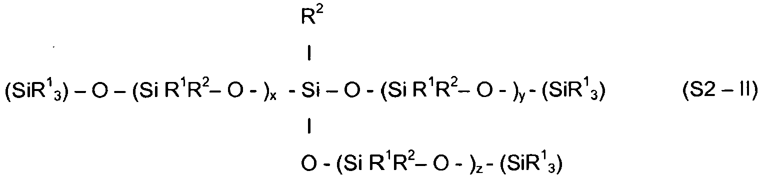

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und besonders bevorzugt ist R¹ und R² Methyl. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, besonders bevorzugte Viskositäten liegen zwischen 10000 und 3000000 cPs. Ganz besonders bevorzugt liegt die Viskosität im Bereich zwischen 50000 und 2000000 cPs. Dimethiconcopolyole (S3) bilden eine weitere Gruppe von Silikonen, die geeignet sind. Dimethiconcopolyole können durch die folgenden Strukturformeln dargestellt werden:

(SiR¹₃)-O-(SiR²₂-O-)x-(SiR²PE-O-)y-(SiR¹₃) (S3 - I),

PE - (SiR¹₂) - O - (SiR²₂ - O -)ₓ -(SiR¹₂) - PE (S3 - II)

Verzweigte Dimethiconcopolyole können durch die Strukturformel (S3 - III) dargestellt werden: 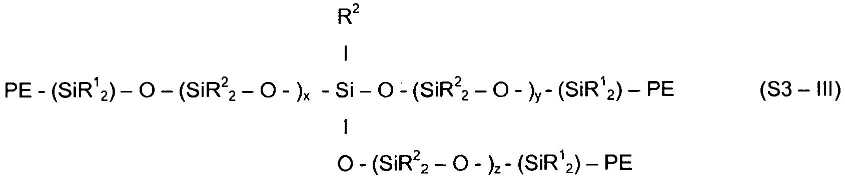 oder durch die Strukturformel (S3 - IV):

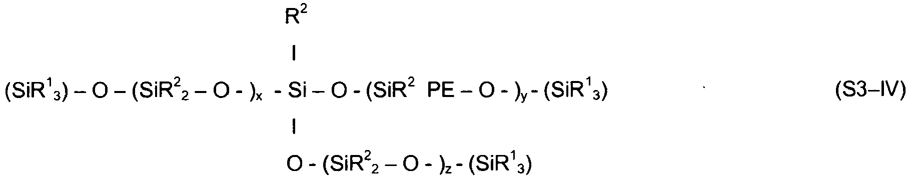

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, insbesondere bevorzugt ist R¹ und R² Methyl. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Vsikositäten liegen zwischen 10000 und 3000000 cPs. Der bevorzugteste Bereich liegt zwischen 50000 und 2000000 cPs.

Entsprechende Dimethiconcopolyole sind kommerziell erhältlich und werden beispielsweise von der Firma Dow Corning unter der Bezeichnung Dow Corning^{®} 5330 Fluid vertrieben.

Selbstverständlich können die Dimethiconole, Dimethicone und/oder Dimethiconcopolymere bereits als Emulsion vorliegen. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Geeignete Silikone sind weiterhin aminofunktionelle Silikone (S4), insbesondere die Silikone, die unter der INCI-Bezeichnung Amodimethicone zusammengefasst sind. Darunter sind Silikone zu verstehen, welche mindestens eine, gegebenenfalls substituierte, Aminogruppe aufweisen.

Solche Silikone können z.B. durch die Formel (S4 - I)

M(RₐQ_{b}SiO_{(4-a-b)/2)})ₓ(R_{c}SiO_{(4-c)/2)})_{y}M (S4 - I)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹Z ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silikon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-,-CH₂CH₂OCH₂-, -OCH₂CH₂-, -OCH₂CH₂CH₂-, -CH₂CH(CH₃)C(O)OCH₂-, -(CH₂)₃C(O)OCH₂CH₂-,-C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z für eine ganze Zahl von 1 bis 50 steht. Eine andere mögliche Formel für Z ist -NH(CH₂)_{z}NH(CH₂)_{zz}, worin sowohl z als auch zz unabhängig voneinander für eine ganze Zahl von 1 bis 50 stehen, wobei diese Struktur Diamino-Ringstrukturen umfasst, wie Piperazinyl. Z ist insbesondere bevorzugt ein -NHCH₂CH₂NH₂-Rest. Eine andere mögliche Formel für Z ist - N(CH₂)_{z}NX¹X² oder -NX¹X², worin X¹ und X² jeweils unabhängig voneinander ausgewählt ist aus Wasserstoff und einem Kohlenwasserstoffrest mit 1 bis etwa 6 Kohlenstoffatomen.

Ganz besonders bevorzugt steht Q für einen polaren, aminfunktionellen Rest der Formel - CH₂CH₂CH₂NHCH₂CH₂NH₂.

Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und besonders bevorzugt von etwa 1 : 15 bis etwa 1 : 20. Werden ein oder mehrere Silikone der obigen Formel eingesetzt, dann können die verschiedenen variablen Substituenten in der obigen Formel bei den verschiedenen Silikonkomponenten, die in der Silikonmischung vorhanden sind, verschieden sein.

Bevorzugte aminofunktionelle Silikone entsprechen der Formel (S4 - II)

R'ₐG₃₋ₐ-Si(OSiG₂)ₙ-(OSiG_{b}R'₂-_{b})ₘ-O-SiG₃₋ₐ-R'a (S4-II),

worin bedeutet:
- G ist -H, eine Phenylgruppe, -OH, -O-CH₃, -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ○ -N(R")-CH₂-CH₂-N(R")₂
   ○ -N(R")₂
   ○ -N⁺(R")₃A⁻
   ○ -N⁺H(R")₂A⁻
   ○ -N⁺H₂(R")A⁻
   ○ -N(R")-CH₂-CH₂-N⁺R"H₂A⁻,
   wobei jedes R" für gleiche oder verschiedene Reste aus der Gruppe -H,-Phenyl, -Benzyl, der C₁₋₂₀-Alkylreste, vorzugsweise -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, steht und A⁻ ein Anion repräsentiert, welches
   vorzugsweise ausgewählt ist aus Chlorid, Bromid, lodid oder Methosulfat.

Besonders bevorzugte aminofunktionelle Silikone entsprechen der Formel (S4 - III) 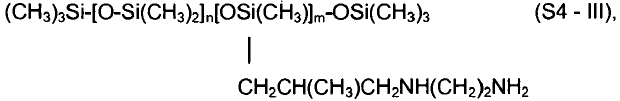 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet.

Besonders bevorzugt sind weiterhin aminofunktionelle Silikone der Formel (S4 - IV)

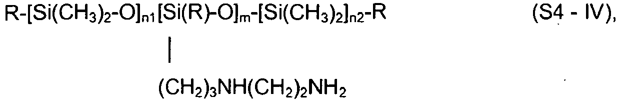

worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone bezeichnet und sind beispielsweise in Form einer Emulsion als Handelsprodukt Dow Corning^{®} 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Weitere geeignete Silikone sind beispielsweise
- oligomere Polydimethylcyclosiloxane (INCI-Bezeichnung: Cyclomethicone), insbesondere die tetramere und die pentamere Verbindung, die als Handelsprodukte DC 245 Fluid, DC 344 bzw. DC 345 von Dow Corning vertrieben werden,
- Hexamethyl-Disiloxan (INCI-Bezeichnung: Hexamethyldisiloxane), z. B. das unter der Bezeichnung Abil^{®} K 520 vertriebenen Produkt,
- Polyphenylmethylsiloxane (INCI-Bezeichnung: Phenyl Trimethicone), z. B. das Handelsprodukt DC 556 Cosmetic Grade Fluid von Dow Corning,
- Ester sowie Partialester der Silikon-Glykol-Copolymere, wie sie beispielsweise von der Firma Fanning unter der Handelsbezeichnung Fancorsil^{®} LIM (INCI-Bezeichnung: Dimethicone Copolyol Meadowfoamate) vertrieben werden,
- anionische Silikonöle, wie beispielsweise das Produkt Dow Corning^{®}1784.

Gemäß einer bevorzugten Ausführungsform enthält das erfindungsgemäße Mittel mindestens zwei unterschiedliche Silikonderivate, insbesondere bevorzugt eine Kombination aus einem flüchtigen und einem nichtflüchtigen Silikon. Flüchtig im Sinne der Erfindung sind solche Silikone, die eine Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning^{®}1401, Dow Corning^{®}1403 und Dow Corning^{®}1501, jeweils Mischungen aus einem Cyclomethicone und einem Dimethiconol) erhältlich.

Bevorzugte Mischungen verschiedener Silikone sind beispielsweise Dimethicone und Dimethiconole, lineare Dimethicone und cylische Dimethiconole. Eine ganz besonders bevorzugte Mischung von Silikonen besteht aus mindestens einem cyclischen Dimethiconol und/oder Dimethicon, mindestens einem weiteren nicht cylischen Dimethicon und/oder Dimethiconol sowie mindestens einem aminofunktionellen Silikon.

Werden unterschiedliche Silikone als Mischung verwendet, so ist das Mischungsverhältnis weitgehend variabel. Bevorzugt werden jedoch alle zur Mischung verwendeten Silikone in einem Verhältnis von 5 : 1 bis 1 : 5 im Falle einer binären Mischung eingesetzt. Ein Verhältnis von 3 : 1 bis 1 : 3 ist besonders bevorzugt. Ganz besonders bevorzugte Mischungen enthalten alle in der Mischung enthaltenen Silikone weitestgehend in einem Verhältnis von etwa 1 : 1, jeweils bezogen auf die eingesetzten Mengen in Gew.-%.

Die Mittel enthalten die Silikone bevorzugt in Mengen von 1 - 25 Gew.-%, besonders bevorzugt von 5 - 20 Gew.-% und insbesondere bevorzugt von 7 - 15 Gew.-%, bezogen auf das gesamte Mittel.

Obwohl das erfindungsgemäße Mittel als Pflegestoff vorzugsweise ein Silikonderivat enthält, ist es auch möglich, dass das Mittel statt oder neben einer Silikonkomponente mindestens einen Pflegestoff einer anderen Verbindungsklasse enthält.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Besonders interessant ist der Einsatz von Seiden-Proteinhydrolysaten. Unter Seide versteht man die Fasern des Kokons des Maulbeer-Seidenspinners (Bombyx mori L.). Die Rohseidenfaser besteht aus einem Doppelfaden Fibroin. Als Kittsubstanz hält Sericin diesen Doppelfaden zusammen. Seide besteht zu 70 - 80 Gew.-% aus Fibroin, 19 - 28 Gew.-% Sericin, 0,5 - 1 Gew.-% aus Fett und 0,5 - 1 Gew.-% aus Farbstoffen und mineralischen Bestandteilen.

Die wesentlichen Bestandteile des Sericin sind mit ca. 46 Gew.-% Hydroxyaminosäuren. Das Sericin besteht aus einer Gruppe von 5 bis 6 Proteinen. Die wesentlichen Aminosäuren des Sericines sind Serin (Ser, 37 Gew.-%), Aspartat (Asp, 26 Gew.-%), Glycin (Gly, 17 Gew.-%), Alanin (Ala), Leucin (Leu) und Tyrosin (Tyr).

Das wasserunlösliche Fibroin ist zu den Skleroproteinen mit langkettiger Molekülstruktur zu zählen. Die Hauptbestandteile des Fibroin sind Glycin (44 Gew.-%), Alanin (26 Gew.-%), und Tyrosin (13 Gew.-%). Ein weiteres wesentliches Strukturmerkmal des Fibroins ist die Hexapeptidsequenz Ser-Gly-Ala-Gly-Ala-Gly.

Technisch ist es auf einfache Art und Weise möglich, die beiden Seidenproteine voneinander zu trennen. So verwundert es nicht, dass sowohl Sericin als auch Fibroin als Rohstoffe zur Verwendung in kosmetischen Produkten jeweils für sich allein bekannt sind. Weiterhin sind Proteinhydrolysate und -derivate auf der Basis der jeweils einzelnen Seidenproteine bekannte Rohstoffe in kosmetischen Mitteln. So wird beispielsweise Sericin als solches seitens der Fa. Pentapharm Ltd. als Handelsprodukt mit der Bezeichnung Sericin Code 303-02 vertrieben. Weitaus häufiger noch wird Fibroin als Proteinhydrolysat mit unterschiedlichen Molekulargewichten im Markt angeboten. Diese Hydrolysate werden insbesondere als "Seidenhydroylsate" vertrieben. So wird beispielsweise unter der Handelsbezeichnung Promois^{®} Silk hydrolysiertes Fibroin mit mittleren Molekulargewichten zwischen 350 und 1000 vertrieben.

Die positiven Eigenschaften der Seidenproteinderivate aus Sericin und Fibroin sind jeweils für sich genommen in der Literatur bekannt. So beschreibt die Verkaufsbroschüre der Fa. Pentapharm die kosmetischen Effekte des Sericines auf der Haut als reizlindernd, hydratisierend und filmbildend. Die Wirkung eines Fibroinderivates wird beispielsweise in der DE 31 39 438 A1 als pflegend und avivierend für das Haar beschrieben. Gemäß DE 102 40 757 A1 lässt sich bei einer gleichzeitigen Verwendung von Sericin und Fibroin bzw. deren Derivaten und/oder Hydrolysaten darüber hinaus eine synergistische Steigerung der positiven Wirkungen der Seidenproteine und deren Derivate erzielen.

Bevorzugt wird daher im erfindungsgemäßen Mittel als Seiden-Proteinhydrolysat ein Wirkstoffkomplex (A) bestehend aus dem Wirkstoff (A1) ausgewählt aus Sericin, Sericinhydrolysaten und/oder deren Derivaten, sowie Mischungen hieraus, und einem Wirkstoff (A2) ausgewählt aus Fibroin, und/oder Fibroinhydrolysaten und/oder deren Derivaten und/oder Mischungen hieraus eingesetzt.

Der Wirkstoffkomplex (A) verbessert signifikant in synergistischer Weise die zuvor dargestellten wesentlichen inneren und äußeren Strukturmerkmale und die Festigkeit sowie die Elastizität von menschlichen Haaren.

Als Wirkstoffe (A1) können im Wirkstoffkomplex (A) verwendet werden:
- natives Sericin,
- hydrolysiertes und/oder weiter derivatisiertes Sericin, wie beispielsweise Handelsprodukte mit den INCI - Bezeichnungen Sericin, Hydrolyzed Sericin, oder Hydrolyzed Silk,
- eine Mischung aus den Aminosäuren Serin, Aspartat und Glycin und/oder deren Methyl-, Propyl-, iso-Propyl-, Butyl-, iso-Butylestern, deren Salze wie beispielsweise Hydrochloride, Sulfate, Acetate, Citrate, Tartrate, wobei in dieser Mischung das Serin und/oder dessen Derivate zu 20 bis 60 Gew.%, das Aspartat und/oder dessen Derivate zu 10 - 40 Gew.% und das Glycin und/oder dessen Derivate zu 5 bis 30 Gew.% enthalten sind, mit der Maßgabe, dass sich die Mengen dieser Aminosäuren und/oder deren Derivate vorzugsweise zu 100 Gew.% ergänzen,
- sowie deren Mischungen.

Als Wirkstoffe (A2) können im Wirkstoffkomplex (A) verwendet werden:
- natives, in eine lösliche Form überführtes Fibroin,
- hydrolysiertes und/oder weiter derivatisiertes Fibroin, besonders teilhydrolisiertes Fibroin, welches als Hauptbestandteil die Aminosäuresequenz Ser-Gly-Ala-Gly-Ala-Gly enthält,
- die Aminosäuresequenz Ser-Gly-Ala-Gly-Ala-Gly,
- eine Mischung der Aminosäuren Glycin, Alanin und Tyrosin und/oder deren Methyl-, Propyl-, iso-Propyl-, Butyl-, iso-Butylestern, deren Salze wie beispielsweise Hydrochloride, Sulfate, Acetate, Citrate, Tartrate, wobei in dieser Mischung das Glycin und/oder dessen Derivate in Mengen von 20 - 60 Gew.%, das Alanin und dessen Derivate in Mengen von 10 - 40 Gew,% und das Tyrosin und dessen Derivate in Mengen von 0 bis 25 Gew.% enthalten sind, mit der Maßgabe, dass sich die Mengen dieser Aminosäuren und/oder deren Derivate vorzugsweise zu 100 Gew.-% ergänzen,
- sowie deren Mischungen.

Besonders gute pflegende Eigenschaften können erzielt werden, wenn eine der beiden Wirkstoffkomponenten des Wirkstoffkomplexes (A) in der nativen oder allenfalls löslich gemachten Form verwendet wird. Es ist auch möglich, eine Mischung aus mehreren Wirkstoffen (A1) und/oder (A2) einzusetzen.

Es kann bevorzugt sein, dass die beiden Wirkstoffe (A1) und (A2) im Verhältnis von 10:90 bis 70:30, insbesondere 15:85 bis 50:50 und ganz besonders 20:80 bis 40:60, bezogen auf deren jeweilige Gehalte an aktiver Wirksubstanz in den erfindungsgemäßen Mitteln eingesetzt werden.

Die Derivate der Hydrolysate von Sericin und Fibroin umfassen sowohl anionische als auch kationisierte Proteinhydrolysate. Die Proteinhydrolysate von Sericin und Fibroin sowie die daraus hergestellten Derivate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche Proteinhydrolysate von Sericin und Fibroin und/oder deren Derivate, deren zugrunde liegender Proteinanteil ein Molekulargewicht von 100 bis zu 25000 Dalton, bevorzugt 250 bis 10000 Dalton aufweist. Weiterhin sind unter kationischen Proteinhydrolysaten von Sericin und Fibroin auch quaternierte Aminosäuren und deren Gemische zu verstehen. Die Quaternisierung der Proteinhydrolysate oder der Aminosäuren wird häufig mittels quartärer Ammoniumsalze wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäß einsetzbaren kationischen Proteinhydrolysate und-derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Cocodimonium Hydroxypropyl Hydrolyzed Silk, Cocodimonium Hydroxypropyl Silk Amino Acids, Hydroxyproypltrimonium Hydrolyzed Silk, Lauryldimonium Hydroxypropyl Hydrolyzed Silk, Steardimonium Hydroxypropyl Hydrolyzed Silk, Quaternium-79 Hydrolyzed Silk. Als typische Beispiele für die erfindungsgemäßen anionischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte genannt: Potassium Cocoyl Hydrolyzed Silk, Sodium Lauroyl Hydrolyzed Silk oder Sodium Stearoyl Hydrolyzed Silk. Letztlich seien noch als typische Beispiele für die erfindungsgemäß einsetzbaren Derivate aus Sericin und Fibroin die unter den INCI - Bezeichnungen im Handel erhältlichen Produkte genannt: Ethyl Ester of Hydrolyzed Silk und Hydrolyzed Silk PG-Propyl Methylsilanediol. Weiterhin erfindungsgemäß verwendbar, wenngleich nicht unbedingt bevorzugt sind die im Handel erhältlichen Produkte mit den INCI - Bezeichnungen Palmitoyl Oligopeptide, Palmitoyl Pentapeptide-3, Palmitoyl Pentapeptide-2, Acetyl Hexapeptide-1, Acetyl Hexapeptide-3, Copper Tripeptide-1, Hexapeptide-1, Hexapeptide-2, MEA-Hydrolyzed Silk.

Die Wirkung des Wirkstoffkomplexes (A) kann durch die Zugabe von Fettstoffen weiter gesteigert werden. Unter Fettstoffen sind zu verstehen Fettsäuren, Fettalkohole, natürliche und synthetische Wachse, welche sowohl in fester Form als auch flüssig in wässriger Dispersion vorliegen können, und natürliche und synthetische kosmetische Ölkomponenten zu verstehen.

Proteinhydrolysate pflanzlichen Ursprungs, z. B. Soja-, Mandel-, Erbsen-, Kartoffel- und Weizenproteinhydrolysate, sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans-Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff einer anderen Verbindungsklasse sind weiterhin kationische Tenside geeignet. Neben den Silikonölen und/oder Silikongummis sind die kationischen Tenside eine bevorzugte Klasse an Pflegestoffen.

Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. Es sei an dieser Stelle darauf hingewiesen, dass einige pflegende Polymere auch filmbildende und/oder festigende Eigenschaften aufweisen, und daher auch als filmbildende und/oder festigende Polymere eingesetzt werden können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (G1-I), 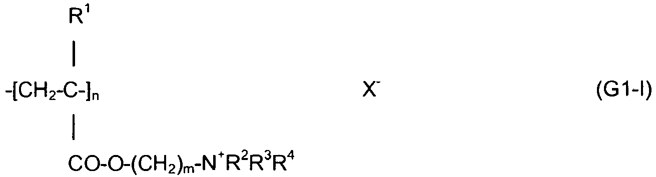 in der R¹= -H oder -CH₃ ist, R², R³ und R⁴ unabhängig voneinander ausgewählt sind aus C₁₋₄-Alkyl-, -Alkenyl- oder-Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliche Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(meth-acryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer^{®} JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI-Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus
(a) Monomeren mit quartären Ammoniumgruppen der allgemeinen Formel (II),

   R¹-CH=CR²-CO-Z-(CₙH₂ₙ)-N⁽⁺⁾R³R⁴R⁵ A⁽⁻⁾ (II)

   in der R¹ und R² unabhängig voneinander stehen für Wasserstoff oder eine Methylgruppe und R³, R⁴ und R⁵ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (III),

   R⁶-CH=CR⁷-COOH (III)
in denen R⁶ und R⁷ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyltrimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte Anwendungszubereitung, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf die gesamte Anwendungszubereitung, enthalten. Mengen von 0,1 - 5 Gew.-% sind besonders bevorzugt.
- Vitamin B₆ (Pyridoxin sowie Pyridoxamin und Pyridoxal). Die genannten Verbindungen des Vitamin B₆-Typs sind in den erfindungsgemäßen Mitteln bevorzugt in Mengen von 0,01 - 5 Gew.-%, bezogen auf die gesamte Anwendungszubereitung, enthalten. Mengen von 0,05 - 1 Gew.-% sind besonders bevorzugt.

Vitamin C (Ascorbinsäure). Vitamin C wird in den erfindungsgemäßen Mitteln bevorzugt in Mengen von 0,1 bis 3 Gew.-%, bezogen auf die gesamte Anwendungszubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf die gesamte Anwendungszubereitung, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6a*R*)2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-%, jeweils bezogen auf die gesamte Anwendungszubereitung enthalten.

Bevorzugt enthalten die erfindungsgemäßen Mittel Vitamine, Provitamine und Vitaminvorstufen aus den Gruppen A, B, C, E und H.

Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Als Pflegestoff können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders geeignet sind die Extrakte aus Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Als Pflegestoff eignet sich weiterhin eine Reihe von Carbonsäuren.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäß einsetzbaren Carbonsäuren können selbstverständlich entlang der Kohlenstoffkette oder des Ringgerüstes substituiert sein. Zu den Substituenten der erfindungsgemäß einsetzbaren Carbonsäuren sind beispielsweise zu zählen C₁-C₈-Alkyl-, C₂-C₈-Alkenyl-, Aryl-, Aralkyl- und Aralkenyl-, Hydroxymethyl-, C₂-C₈-Hydroxyalkyl-,C₂-C₈-Hydroxyalkenyl-, Aminomethyl-, C₂-C₈-Aminoalkyl-, Cyano-, Formyl-, Oxo-, Thioxo-, Hydroxy-, Mercapto-, Amino-, Carboxy- oder Iminogruppen. Bevorzugte Substituenten sind C₁-C₈-Alkyl-, Hydroxymethyl-, Hydroxy-, Amino- und Carboxygruppen. Besonders bevorzugt sind Substituenten in α- Stellung. Ganz besonders bevorzugte Substituenten sind Hydroxy-, Alkoxy-und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäß einsetzbare Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, lsonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I), 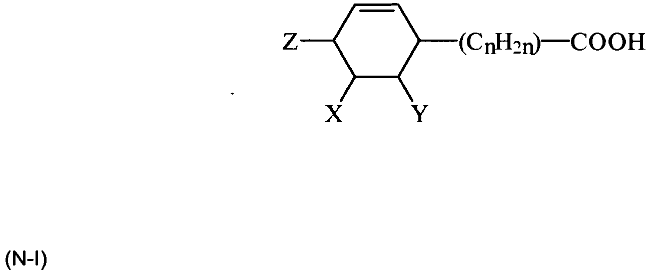 in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt. So ist beispielweise US-A 3,753,968 ein Herstellungsverfahren zu entnehmen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuss vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbindung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei C₁-bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf die gesamte Anwendungszubereitung, besonders bevorzugt 0,1 bis 5 Gew.%, und insbesondere bevorzugt 0,1 bis 3 Gew.%.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, dass neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Als Pflegestoff eignen sich weiterhin Ectoin oder Ectoinderivate, Allantoin, Taurin und/oder Bisabolol.

Erfindungsgemäß werden unter dem Begriff "Ectoin und Ectoinderivate" Verbindungen der Formel (IV) 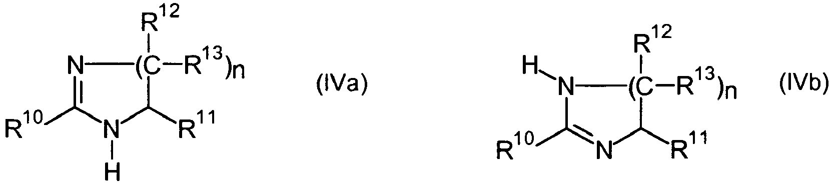 und/oder deren physiologisch verträglichen Salze und/oder eine isomere oder stereoisomere Form verstanden, wobei
R¹⁰ steht für ein Wasserstoffatom, einen verzweigten oder unverzweigten C₁-C₄-Alkylrest oder einen C₂-C₄-Hydroxyalkylrest,
R¹¹ steht für ein Wasserstoffatom, eine Gruppierung -COOR¹⁴ oder eine Gruppierung-CO(NH)R¹⁴, wobei R¹⁴ für ein Wasserstoffatom, einen C₁-C₄-Alkylrest, einen Aminosäurerest, einen Dipeptid- oder einen Tripeptidrest stehen kann,
R¹² und R¹³ stehen unabhängig voneinander für ein Wasserstoffatom, einen C₁-C₄-Alkylrest oder eine Hydroxygruppe mit der Maßgabe, dass nicht beide Reste gleichzeitig für eine Hydroxygruppe stehen dürfen, und
n steht für eine ganze Zahl von 1 bis 3.

Geeignete physiologisch verträgliche Salze der allgemeinen Verbindungen gemäß der Formel (IVa) oder (IVb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IVa) oder (IVb) mit anorganischen und organischen Säuren wie Salzsäure, Phosphorsäure, Schwefelsäure, verzweigten oder unverzweigten, substituierten oder unsubstituierten (beispielsweise durch eine oder mehrere Hydroxygruppen) C₁-C₄- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IVa) oder (IVb), sowie die Salze, die sich durch Umsetzung von Verbindungen gemäß der Formel (IVa) oder (IVb) mit Salzsäure, Essigsäure, Citronensäure und Benzoesäure ergeben.

Unter isomeren oder stereoisomeren Formen der Verbindungen gemäß Formel (IVa) oder (IVb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden:
Asparagin, Arginin, Asparaginsäure, Glutamin, Glutaminsäure, β-Alanin, γ-Aminobutyrat, N_{ε}Acetyllysin, N_{ô}-Acetylornitin, N_{γ}-Acetyldiaminobutyrat, N_{α}-Acetyldiaminobutyrat, Histidin, Isoleucin, Leucin, Methionin, Phenylalanin, Serin, Threonin und Tyrosin.
L-Aminosäuren sind bevorzugt. Aminosäurereste leiten sich von den entsprechenden Aminosäuren ab. Die folgenden Aminosäurereste sind bevorzugt:
Gly, Ala, Ser, Thr, Val, β-Ala, γ-Aminobutyrat, Asp, Glu, Asn, Aln, N_{ε}-Acetyllysin, N_{δ}-Acetylornithin, N_{γ}-Acetyldiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für C₁ - C₄-Alkylgruppen in den Verbindungen der Formel (IV) sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte C₂ - C₄-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Die erfindungsgemäßen Mittel enthalten diese Pflegestoffe bevorzugt in Mengen von 0,001 bis 2, insbesondere von 0,01 bis 0,5 Gew.-%, jeweils bezogen auf die gesamte Anwendungszubereitung.

Auch Mono- bzw. Oligosaccharide können als Pflegestoff in den erfindungsgemäßen Mitteln eingesetzt werden.

Es können sowohl Monosaccharide als auch Oligosaccharide, wie beispielweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCl-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Die erfindungsgemäßen Mittel enthalten die Lipide bevorzugt in Mengen von 0,01 - 10 Gew.-%, insbesondere 0,1 - 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀-Fettsäuren mit C₂ - C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 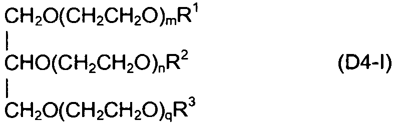
in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Das Mittel kann überdies ein Enzym als Pflegestoff enthalten. Erfindungsgemäß besonders bevorzugte Enzyme sind ausgewählt aus einer Gruppe, die gebildet wird aus Proteasen, Lipasen, Transglutaminase, Oxidasen und Peroxidasen.

Auch Perlenextrakte sind als Pflegestoff geeignet.

Perlen von Muscheln bestehen im Wesentlichen aus anorganischen und organischen Calciumsalzen, Spurenelementen und Proteinen. Perlen lassen sich auf einfache Weise aus kultivierten Muscheln gewinnen. Die Kultivierung der Muscheln kann sowohl in Süßwasser als auch in Meereswasser erfolgen. Dies kann sich auf die Inhaltsstoffe der Perlen auswirken. Erfindungsgemäß bevorzugt ist ein Perlenextrakt, welcher von in Meeres- bzw. Salzwasser kultivierten Muscheln stammt. Die Perlen bestehen zu einem großen Teil aus Aragonit (Calciumcarbonat), Conchiolin und einem Albuminoid. Letztere Bestandteile sind Proteine. Weiterhin sind in Perlen noch Magnesium- und Natriumsalze, anorganische Siliciumverbindungen sowie Phosphate enthalten.

Zur Herstellung des Perlenextraktes werden die Perlen pulverisiert. Danach werden die pulverisierten Perlen mit den üblichen Methoden extrahiert. Als Extraktionsmittel zur Herstellung der Perlenextrakte können Wasser, Alkohole sowie deren Mischungen verwendet werden. Unter Wasser sind dabei sowohl demineralisiertes Wasser, als auch Meereswasser zu verstehen. Unter den Alkoholen sind dabei niedere Alkohole wie Ethanol und Isopropanol, insbesondere aber mehrwertige Alkohole wie Glycerin, Diglycerin, Triglycerin, Polyglycerin, Ethylenglykol, Propylenglykol und Butylenglykol, sowohl als alleiniges Extraktionsmittel als auch in Mischung mit demineralisiertem Wasser oder Meereswasser, bevorzugt. Perlenextrakte auf Basis von Wasser/Glyceringemischen haben sich als besonders geeignet erwiesen. Je nach Extraktionsbedingungen können die Perlenproteine (Conchiloin und Albuminoid) weitestgehend in nativem Zustand oder bereits teilweise oder weitestgehend als Proteinhydrolysate vorliegen. Bevorzugt ist ein Perlenextrakt, in welchem Conchiolin und Albuminoid bereits teilweise hydrolysiert vorliegen. Die wesentlichen Aminosäuren dieser Proteine sind Glutaminsäure, Serin, Alanin, Glycin, Asparaginsäure und Phenylalanin. In einer weiteren besonders bevorzugten Ausgestaltung kann es vorteilhaft sein, wenn der Perlenextrakt zusätzlich mit mindestens einer oder mehreren dieser Aminosäuren angereichert wird. In der bevorzugtesten Ausführungsform ist der Perlenextrakt angereichert mit Glutaminsäure, Serin und Leucin. Weiterhin findet sich je nach Extraktionsbedingungen, insbesondere in Abhängigkeit von der Wahl des Extraktionsmittels ein mehr oder weniger großer Anteil an Mineralien und Spurenelementen im Extrakt wieder. Ein bevorzugter Extrakt enthält organische und/oder anorganische Calciumsalze sowie Magnesium- und Natriumsalze, anorganische Siliciumverbindungen und/oder Phosphate. Ein ganz besonders bevorzugter Perlenextrakt enthält mindestens 75 %, bevorzugt 85 %, besonders bevorzugt 90 % und ganz besonders bevorzugt 95 % aller Inhaltsstoffe der natürlich vorkommenden Perlen. Beispiele für erfindungsgemäß einsetzbare Perlenextrakte sind die Handelsprodukte Pearl Protein Extract BG^{®} oder Crodarom^{®} Pearl.

Die zuvor beschriebenen Perlenextrakte sind vorzugsweise in einer Menge von mindestens 0,01 bis zu 20 Gew.-% enthalten. Bevorzugt werden Mengen des Extraktes von 0,01 bis zu 10 Gew.-%, ganz besonders bevorzugt Mengen von 0,01 bis 5 Gew.-% bezogen auf die gesamte Anwendungszubereitung verwendet.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelte Haut oder die Haare vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbare UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxy-benzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Tri-anilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20 000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein. Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.

Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylaminobenzoesäureamid ableiten, sind erfindungsgemäß bevorzugt.

Die Strukturteile U können prinzipiell so gewählt werden, dass das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, dass der molare Extinktionskoeffizient des UV-Filters am Absorptionsmaximum oberhalb von 15 000, insbesondere oberhalb von 20 000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so dass der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)_{X}-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In Abhängigkeit von der Art des erfindungsgemäßen Mittels kann es erforderlich sein, dass diese weiterhin mindestens ein Tensid enthalten. Dies gilt insbesondere für Shampoos. Aber auch andere Mittel, wie beispielsweise Haarspülungen, Haarkuren, Haarwasser, Haarcreme, Haarlotionen, Haartinkturen, und bestimmte Stylingmittel, insbesondere Stylingschäume, können Tenside enthalten.

Beispielsweise können kationische Tenside eingesetzt werden, wie sie bereits oben als geeignete Pflegestoffe beschrieben sind. Bezüglich der bevorzugten kationischen Tenside und der eingesetzten Mengen gelten obige Ausführungen entsprechend.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

In einer bevorzugten Ausführungsform der vorliegenden Erfindung wird, beispielsweise in einem Shampoo, eine Kombination aus anionischen und nichtionischen Tensiden oder eine Kombination aus anionischen und amphoteren Tensiden eingesetzt. In einem Haartonic kann der Fachmann jedoch auch weitgehend oder vollständig auf den Einsatz von Tensiden verzichten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 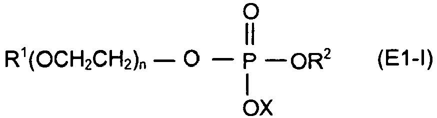 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4-Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III) 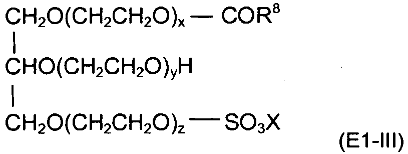 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammo-niumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCl-Bezeichnung Cocamidopropyl Betaine bekannte Fettsäureamid-Derivat.

Unter Ampholyten werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)
in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀(DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 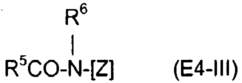 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

Die Mittel können weiterhin mindestens einen Emulgator enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 100 Mol Ethylenoxid und/oder 1 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- lineare und verzweigte Fettsäuren mit 8 bis 30 C - Atomen und deren Na-, K-, Ammonium-, Ca-, Mg- und Zn - Salze.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 sind erfindungsgemäß besonders bevorzugt.

Es kann weiterhin erforderlich sein, dass die erfindungsgemäßen Mittel weiterhin mindestens ein Verdickungsmittel enthalten. Dies trifft insbesondere dann zu, wenn es sich bei dem Mittel um ein Gel oder eine Paste handelt.

Geeignete Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate. Auch die Polymere, die als mögliche Pflegestoffe beschrieben wurden oder im Folgenden als filmbildende und/oder festigende Polymere aufgezählt werden, können verdickende Eigenschaften aufweisen, und können gegebenenfalls als Verdickungsmittel Verwendung finden.

Die Mittel können neben den genannten Komponenten weiterhin alle für entsprechende kosmetische Mittel bekannten Wirk-, Zusatz- und Hilfsstoffe enthalten. Dies sind beispielsweise
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für kosmetische Mittel üblichen Formen erfolgen, beispielsweise in Form von Lösungen, die als Haarwasser oder Pump- oder Aerosolspray auf das Haar aufgebracht werden können, in Form von Emulsionen, Wachsen, Gelen oder auch tensidhaltigen schäumenden Lösungen oder anderen Zubereitungen, die für die Anwendung auf dem Haar geeignet sind.

In einer bevorzugten Ausführungsform handelt es sich bei den erfindungsgemäßen Mitteln um Haarbehandlungsmittel, insbesondere Shampoo, Haarnachspülmittel, Haargel, Haarwasser, Haarkur, Haarcreme, Haarlotion, Haarspray und Haartinktur.

Die erfindungsgemäßen Mittel können beispielsweise als Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole formuliert vorliegen. Die Mittel können als Einkammersystem oder als Zweikammersystem konfektioniert werden.

Erfindungsgemäß von besonderem Interesse sind Haartonics, insbesondere als auf dem Haar verbleibende (leave on) Formulierung. Diese werden vorzugsweise bei Raumtemperatur angewendet, der alkoholische Gehalt liegt bevorzugtermaßen im Bereich von etwa 30 % bis etwa 35 % und der pH-Wert sollte etwa bei pH 7 liegen. Insbesondere bei Haartonics hat sich der Einsatz von in Liposomen verkapseltem Purin und / oder Purinderivate und Taurin als vorteilhaft erwiesen. Die Verkapselung der Wirkstoffe, mit anschließender Verkapselung in Liposomen ist besonders bevorzugt. Solche Liposomen können insbesondere in Haartonics eingesetzt werden. In den verkapselten Liposomen können neben Purin und / oder Purinderivate und Taurin weitere Substanzen eingekapselt sein, die für die Anwendung zweckmäßig sind.

In einer besonders bevorzugten Ausführungsform handelt es sich bei den erfindungsgemäßen Mitteln um Mittel zur temporären Verformung keratinischer Fasern, d.h. um Stylingmittel.

Diese enthalten als weitere Komponente mindestens ein filmbildendes und/oder festigendes Polymer. In einer besonderen Ausführungsform enthalten die erfindungsgemäßen Mittel daher bezogen auf das gesamte Mittel 0,01 bis 20 Gew.-% mindestens eines filmbildenden und/oder festigenden Polymers. Besonders bevorzugt wird das filmbildende und/oder festigende Polymer in einer Menge von 0,5 bis 15 Gew.-%, und ganz besonders bevorzugt von 1 bis 10 Gew.-%, jeweils bezogen auf das gesamte Mittel, eingesetzt. Selbstverständlich können auch mehrere filmbildende und/oder festigende Polymere eingesetzt werden.

Die Formulierung kosmetischer Mittel, die der Verminderung von vorzeitigem Haarausfall und/oder der Förderung des Haarwachstums dienen sollen, als Stylingmittel ist ungewöhnlich, da zunächst zu erwarten ist, dass Stylingmittel bei der bestimmungsgemäßen Anwendung kaum mit der Kopfhaut in Kontakt kommen und damit die notwendige Wechselwirkung zwischen Wirkstoff und Haarfollikel nicht gewährleistet ist. Dies ist jedoch überraschenderweise nicht der Fall, wie folgende Untersuchung zeigt.

Die Haare vierer Puppenköpfe, wie sie üblicherweise in Haarstudios zu Übungs- und Testzwecken eingesetzt werden, werden zunächst so geschnitten, dass eine Länge resultiert, bei der üblicherweise Stylinggele zur Anwendung kommen. Die Haarlänge im Bereich des Oberkopfes beträgt etwa 5 cm. Ein erfahrener Friseur bringt anschließend die Haare unter Anwendung herkömmlicher Stylinggele, denen Fluoreszenzfarbstoffe zugemischt wurden, in Form. Die Gele werden dabei entweder gezielt einmassiert oder auf herkömmliche Art und Weise angewendet. Nach erfolgter Behandlung werden die Haare trocken auf eine Länge von etwa 1 mm rasiert, Haarreste abgeföhnt und anschließend der Puppenkopf unter Schwarzlicht betrachtet. Anhand des Aufleuchtens des Fluoreszenzfarbstoffes lassen sich die Stellen des Puppenkopfes, die mit dem jeweiligen Stylinggel in Kontakt gekommen sind, optisch leicht detektieren. Es zeigt sich, dass unabhängig vom untersuchten Stylinggel sowohl beim Einmassieren, als auch bei der üblichen Anwendung des Gels ein deutlicher Kontakt zwischen Stylinggel und Puppenkopf stattfindet. Das Einmassieren führt dabei zu keiner sichtbaren Verbesserung des Kontakts.

Die Formulierung kosmetischer Mittel, die der Verminderung von vorzeitigem Haarausfall und/oder der Förderung des Haarwachstums dienen sollen, als Stylingmittel hat verschiedene Vorteile. Zum einen handelt es sich bei Stylingmitteln in der Regel um sogenannte Leave-on Produkte, d.h. Produkte, die nach der Anwendung für einen längeren Zeitraum im Haar verbleiben. Im Vergleich zu anderen üblichen Applikationsformen, etwa Shampoos, ist damit die Zeit, in der der Wirkstoff und die Haarfollikel wechselwirken können, deutlich erhöht. Zum anderen dient das Mittel gleichzeitig auch zur Frisurengestaltung und/oder -festigung, d.h. der temporären Verformung der Haare. Der Verbraucher erzielt also bei Anwendung nur eines Mittels mehrere Effekte gleichzeitig, wodurch die Bereitschaft zur regelmäßigen Anwendung des Mittels erhöht wird.

Als filmbildende und/oder festigende Polymere können alle in dieser Funktion bekannten Polymere eingesetzt werden, wobei diese sowohl permanent als auch temporär kationisch, anionisch, nichtionisch oder amphoter sein können. Bei der Verwendung von mindestens zwei filmbildenden und/oder festigenden Polymeren können diese selbstverständlich unterschiedliche Ladungen aufweisen. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft einige filmbildende Polymere beschrieben werden. Es wird an dieser Stelle jedoch explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁-bis C₇-Alkylgruppen, besonders bevorzugt C₁- bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise, unter den Handelsbezeichnungen Ucon^{®} der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird.

Festigende Polymere tragen zum Halt und/oder zum Aufbau des Haarvolumens und der Haarfülle der Gesamtfrisur bei. Diese so genannten festigenden Polymere sind gleichzeitig auch filmbildende Polymere und daher generell typische Substanzen für formgebende Haar-behandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein lang anhaltender Frisurenaufbau und -erhalt gewährleistet. Als Testmethode hierfür wird häufig der so genannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß bevorzugt zu verwendenden Polymere finden sich somit selbstverständlich gerade auch die genannten filmbildenden Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymere, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquatemium-18, Polyquaternium-19, Polyquaternium-20, Polyquaternium-22, Polyquaternium-24, Polyquaternium-27, Polyquaternium-28, Polyquaternium-29, Polyquaternium-30, Polyquatemium-31, Polyquaternium-32, Polyquaternium-33, Polyquaternium-34, Polyquaternium-35, Polyquaternium-36, Polyquaternium-37, Polyquaternium-39, Polyquaternium-45, Polyquatemium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxy-benzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate.

Bevorzugte filmbildende und/oder festigende Polymere sind die Polyvinylpyrrolidone, Vinyl-pyrrolidon-Vinylacetat-Copolymere, Vinylacetat-Crotonsäure-Copolymere, Vinylcaprolactam-Vinylpyrrolidon-Dimethylaminoethylmethacrylat-Copolymere, Octylacrylamid-Acrylat-Butylaminoethyl-Methacrylat-Copolymere und quaternierte Vinylpyrrolidon-Dimethylaminoethylmethacrylat-Copolymere.

Besonders bevorzugt handelt es sich bei dem filmbildenden und/oder festigenden Polymer um die Vinylpyrrolidon-Vinylacetat-Copolymeren Luviskol^{®} VA 37 oder PVPNA Copolymer 60/40 W NP, das Vinylacetat-Crotonsäure-Copolymer, das unter der Handelsbezeichnung Aristoflex^{®} A 60 vertrieben wird, das Vinylcaprolactam-Vinylpyrrolidon-Dimethylaminoethylmethacrylat-Copolymer mit der Handelsbezeichnung Advantage^{®} LC-E, das unter der Bezeichnung Amphomer^{®} erhältliche amphotere Octylacrylamid-Acrylat-Butylaminoethyl-Methacrylat-Copolymer oder das durch Umsetzung mit Diethylsulfat quaternierte Vinylpyrrolidon-Dimethylaminoethylmethacrylat-Copolymer, das unter der Handelsbezeichnung Gafquat^{®} 755N vertrieben wird.

Insbesondere bevorzugt handelt es sich bei dem filmbildenden und/oder festigenden Polymer um ein Vinylpyrrolidon-Vinylacetat-Copolymer.

Bevorzugte erfindungegemäße Stylingmittel sind Stylinggele, Pumphaarsprays, Aerosolhaarsprays, Pumphaarschäume und Aerosolhaarschäume. Besonders bevorzugt handelt es sich bei den erfindungsgemäßen Stylingmitteln um Stylinggele.

Unter Haarschäumen werden dabei Zusammensetzungen verstanden, die bei der Entnahme aus einem geeigneten Behälter einen Schaum ausbilden. Es kann notwendig sein, den Mitteln Inhaltsstoffe zuzusetzen, die die Schaumbildung fördern oder einmal gebildeten Schaum stabilisieren. Insbesondere eignen sich dafür Tenside und/oder Emulgatoren, wie sie bereits oben beschrieben wurden. Vorzugsweise werden Tenside aus der Gruppe der kationischen Tenside eingesetzt.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt. Geeignete und bevorzugte Strukturanten und/oder verdickende Polymere sind bereits oben beschrieben.

Sofern es sich bei den erfindungsgemäßen Mitteln um ein Aerosolprodukt handelt, enthält dieses zwingend ein Treibmittel.

Erfindungsgemäß geeignete Treibmittel sind beispielsweise N₂O, Dimethylether, CO₂, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen.

Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Wenngleich die zuvor genannten Haarbehandlungsmittel erfindungsgemäß bevorzugt sind, kann es sich bei den erfindungsgemäßen Mitteln auch um andere Haarbehandlungsmittel, wie z.B. Haarfärbemittel und Wellmittel, handeln. Diese Mittel enthalten dann gegebenenfalls die bekannten direktziehenden Farbstoffe, Vorläufer für Oxidationsfarbstoffe (Entwickler- und Kupplerkomponenten) und Oxidationsmittel bzw. Reduktionsmittel. Vorteilhafterweise können die erfindungsgemäßen Mittel so das Haar vor der Beanspruchung bei der Färbung schützen, das Haarfollikel aktivieren und gleichzeitig Hautirritationen durch die Well- bzw. Haarfärbemitteln vermindern bzw. lindern.

Ein zweiter Gegenstand der Erfindung ist die Verwendung der erfindungsgemäßen Mittel zur Vitalisierung von Haaren, Reaktivierung der Haarwurzel, Aktivierung von Haarfollikeln, Förderung oder Verstärkung des Haarwachstums, zur Haarkonditionierung und / oder zur Verminderung und / oder Behandlung von Haarausfall.

Ein dritter Gegenstand der Erfindung ist die Verwendung der erfindungsgemäßen Stylingmittel zur Vitalisierung von Haaren, Reaktivierung der Haarwurzel, Aktivierung von Haarfollikeln, Förderung oder Verstärkung des Haarwachstums, zur Haarkonditionierung und / oder zur Verminderung und / oder Behandlung von Haarausfall und zur gleichzeitigen temporären Verformung von Haaren. Bevorzugt ist dabei die Verwendung der erfindungsgemäßen Stylingmittel zur Förderung oder Verstärkung des Haarwachstums und / oder zur Verminderung und / oder Behandlung von Haarausfall und zur gleichzeitigen temporären Verformung von Haaren

Ein weiterer Gegenstand der vorliegenden Erfindung ist ein kosmetisches Verfahren zur Vitalisierung von Haaren, Reaktivierung der Haarwurzel, Aktivierung von Haarfollikeln, Förderung oder Verstärkung des Haarwachstums, zur Haarkonditionierung, zur Verminderung von Haarausfall und / oder zur Behandlung von Haarausfall, wobei die erfindungsgemäßen Mittel auf die Haare, Kopfhaut, behaarte Haut bzw. auf Hautstellen, wo der Haarwuchs seit längerem eingestellt ist, wo jedoch ein Haarwuchs gewünscht wird, aufgebracht wird.

Die nachfolgenden Beispiele sollen den Gegenstand der vorliegenden Erfindung erläutern ohne ihn in irgendeiner Weise zu beschränken.

### Beispiele:

Die folgenden Mengenangaben verstehen sich - soweit nichts anderes vermerkt ist - in Gewichtsprozent.

### 1 Stylinggele

Es wurden die erfindungsgemäßen Stylinggele E1 bis E4 gemäß folgender Tabelle 1 hergestellt.

**Tabelle 1**

| **Rohstoffe** | **E1** | **E2** | **E3** | **E4** |
|---|---|---|---|---|
| Taurin | 0,05 | 0,05 | 0,05 | 0,10 |
| Coffein | 0,10 | 0,10 | 0,10 | 0,05 |
| Carnitintartrat | - | 0,10 | - | - |
| Minoxidil | - | - | 0,20 | - |
| PEG-40 Hydrogenated Castor Oil ¹ | 0,40 | 0,40 | 0,40 | 0,30 |
| Synthalen K ² | 0,50 | 0,50 | 0,50 | - |
| Methylparaben | 0,10 | 0,10 | 0,10 | - |
| PVP/VA W-635 ³ | 5,00 | 5,00 | 5,00 | 7,00 |
| Triethanolamin 99 % | 0,30 | 0,30 | 0,30 | - |
| Luviset Clear ⁴ | 4,50 | 4,50 | 4,50 | - |
| Sorbit 70% | 1,50 | 1,50 | 1,50 | - |
| D-Panthenol 75% | 0,30 | 0,30 | 0,30 | - |
| Tylose H ⁵ | - | - | - | 1,00 |
| Celquat L-200 ⁶ | - | - | - | 0,75 |
| Neutrol TE ⁷ | - | - | - | 0,15 |
| Dow Corning 190 Surfactant ⁸ | - | - | - | 0,10 |
| Nutrilan I 50 ⁹ | - | - | - | 0,30 |
| Extrapon Brennnessel Spezial No. 680826 ¹⁰ | - | - | - | 3,00 |
| Dehyquart SP ¹¹ | - | - | - | 0,10 |
| Natriumbenzoat | - | - | - | 0,10 |
| Parfüm | 0,10 | 0,10 | 0,10 | 0,20 |
| Ethanol 96 %, vergällt | - | - | - | 10,70 |
| Wasser, entsalzt | ad 100 | ad 100 | ad 100 | ad 100 |

| | | | | |
|---|---|---|---|---|
| ¹ Hydriertes Rizinusöl mit ca. 40-45 EO-Einheiten (INCl-Bezeichnung: PEG-40 Hydrogenated Castor Oil) (BASF) ² Polyacrylsäure (ca. 89% Aktivsubstanzgehalt; INCl-Bezeichnung: Carbomer) (3V Sigma) ³ Vinylpyrrolidon Vinylacetat Copolymer (ca. 48-52% Festkörper in Wasser ; INCI-Bezeichnung : VP/VA Copolymer) (ISP) ⁴ Vinylpyrrolidon-Methacrylamid-Vinylimidazol-Copolymerisat (19-21 % Festkörper in Wasser; INCl-Bezeichnung: VP/Methacrylamide/Vinyl Imidazole Copolymer) (BASF) ⁵ Modifizierte Cellulose (90% Aktivsubstanzgehalt; INCl-Bezeichnung: Hydroxyethylcellulose) (Shin Etsu) ⁶ Quaterniertes Cellulose-Derivat (INCl-Bezeichnung: Polyquaternium-4) (National Starch) ⁷ N,N,N',N',-Tetrakis-(2-hydroxypropyl)-ethylendiamin (INCl-Bezeichnung: Tetrahydroxypropyl Ethylenediamine) (BASF) ⁸ Mit durchschnittlich 18 Mol Propylenoxid und 18 Mol Ethylenoxid alkoxyliertes Polydimethylsiloxan-Derivat (INCl-Bezeichnung: PEG/PPG-18/18 Dimethicone) (Dow Corning) ⁹ Collagenproteinhydrolysat (ca. 50-52% Festkörper; INCl-Bezeichnung: Aqua (Water), Hydrolyzed Collagen, Benzoic Acid, Methylparaben, Ethylparaben) (Cognis) ¹⁰ Brennnesselextrakt (4-5% Festkörper; INCl-Bezeichnung: Urtica Dioica (Nettle) Extract) (Dragoco) ¹¹ Tris-(oligooxyethyl)alkylammoniumphosphat (ca. 49-51% Aktivsubstanz in Wasser; INCI-Bezeichnung: Aqua (Water), Quaternium-52, Propylene Glycol) (Cognis) | | | | |

Die Gele wurden durch übliches Vermischen der in der Tabelle genannten Rohstoffe hergestellt.

### 2 Wirknachweise

Zum Nachweis biologischer Effekte von Coffein und Taurin wurden die wässrigen Lösungen gemäß Tabelle 2 an dermalen Papillenzellen untersucht, wobei jede Bestimmung 4-fach durchgeführt wurde.

**Tabelle 2: Untersuchte Lösungen**

| | Substanzkombinationen | |
|---|---|---|
| | Taurin (%) | Coffein (%) |
| Gemisch | 0,5 | 0,025 |
| Taurin | 0,5 | 0 |
| Coffein | 0 | 0,025 |
| Kontrolle | 0 | 0 |

### Beispiel A: Bestimmung zellschädigender Nebeneffekte durch die Behandlung mit Coffein, Taurin und die Kombination beider Substanzen (Coffein und Taurin) in kultivierten dermalen Papillenzellen

Die Bestimmung der Freisetzung von Laktat-Dehydrogenase (LDH) kultivierter Zellen gibt Auskunft über die Schädigung der Zellmembran. In unbeschädigten Zellen ist LDH ausschließlich im Zytoplasma lokalisiert. Erst durch Schädigung der Zellmembran kann LDH in das umgebende Kulturmedium diffundieren. Mit Hilfe eines Substrat-gekoppelten kolorimetrischen Testsystems kann die Menge an freigesetztem LDH bestimmt werden.

Zur Bestimmung möglicher zellschädigender Nebeneffekte wurden kultivierte dermale Papillenzellen mit den in Tabelle 2 aufgeführten Lösungen kultiviert und nach Behandlung die Menge an LDH im Medium analysiert. Es wurde die Zeit bestimmt, die benötigt wird, um eine definierte Menge an Farbstoff umzusetzen. In Tabelle 3 sind die erhaltenen Werte aufgeführt. Hierbei gilt, dass Werte < 80 % auf mögliche zellschädigende Reaktionen hindeuten.

**Tabelle 3: Analyse der Freisetzung von Laktat-Dehydrogenase nach Behandlung mit Coffein, Taurin und deren Mischung**

| | Freisetzung von LDH | |
|---|---|---|
| | Mittelwert (%) | SD (%) |
| Kontrolle | 100 | 7,8 |
| Gemisch | 112,4 | 4,4 |
| Coffein | 126,2 | 5,7 |
| Taurin | 134,1 | 6,2 |

Die Ergebnisse in Tabelle 3 zeigen, dass weder nach Behandlung mit Taurin oder Coffein allein, noch mit einem Gemisch beider Verbindungen zellschädigende Effekte nachgewiesen werden können.

### Beispiel B: Nachweis der differentiellen Expression von Haarzyklus-regulierenden Genen

Hepatocyte Growth Factor (HGF) und Keratinocyte Growth Factor (KGF) sind wichtige Wachstumsfaktoren, die von den dermalen Papillenzellen synthetisiert werden. Im Speziellen stimuliert HGF das Follikelwachstum und KGF die Keratinozytenproliferation. Entsprechend werden HGF und KGF in der Wachstumsphase vermehrt exprimiert.

Diesen Eigenschaften gegenüber steht die Expression von IGFBP-3. Dieses Genprodukt führt neben Anderen zu einer.Verschiebung des Zyklus von der Wachstums- zur Regressionsphase. Nach Applikation von Substanzen, die Effekte am biologisch aktiven Teil des Haares hervorrufen, könnte die Bestimmung der Veränderungen der Expression dieses Gens Aussagen über Haarwuchsstimulantien oder -inhibitoren liefern.

Die differentielle Genexpression wurde mittels quantitatitiver Reverse-Transkription Polymerase Kettenreaktion (qRT-PCR) bestimmt. Nach Anzucht der dermalen Papillenzellen wurden jeweils 4 biologische Replikate für 24 h mit den Lösungen gemäß Tabelle 2 inkubiert. Zur Durchführung der qRT-PCR wird zunächst mit Hilfe des RNeasy Mini Kits der Fa. Qiagen die RNA aus den dermalen Papillenzellen isoliert und mittels reverser Transkription in cDNA umgeschrieben. Bei der anschließenden PCR Reaktion, die mit Hilfe genspezifischer Primer für die jeweiligen haarrelevanten Gene durchgeführt wird und die der Amplifikation der gesuchten Genabschnitte dient, wird die Bildung der PCR-Produkte online über ein Fluoreszenzsignal detektiert. Das Fluoreszenzsignal ist dabei proportional zur Menge des gebildeten PCR-Produktes. Je stärker die Expression eines bestimmten Gens ist, desto größer ist die Menge an gebildetem PCR-Produkt und umso höher ist das Fluoreszenzsignal.

Zur Quantifizierung der Genexpression wird die unbehandelte Kontrolle gleich 1 gesetzt und die Expression der zu bestimmenden Gene darauf bezogen (x-fache Expression). Dabei werden Werte, die größer/gleich der 2-fachen Expression oder kleiner/gleich der 0,5-fachen Expression der unbehandelten Kontrolle sind als signifikant differentiell exprimiert eingestuft. Werte die größer/gleich der 1,5-fachen Expression oder kleiner/gleich der 0,6-fachen Expression der unbehandelten Kontrolle sind, werden als tendenziell differentiell exprimiert eingestuft. Die Ergebnisse sind in Tabelle 4 zusammengefasst.

**Tabelle 4: Einfluss von Coffein (0,025%), Taurin (0,5%) oder einem Gemisch dieser Substanzen auf die Expression Haarzyklus-regulierender Gene**

| | **HGF** | **KGF** | **IGFBP3** |
|---|---|---|---|
| | Mittelwert | Mittelwert | Mittelwert |
| **Kontrolle** | 1,00 (0,25) | 1,00 (0,13) | 1,00 (0,10) |
| **Gemisch** | 3,30 (0,80) | 1,57(0,14) | 0,42 (0,08) |
| **Taurin** | 1,03 (0,30) | 1,08 (0,12) | 1,41 (0,18) |
| **Coffein** | 1,62 (0,46) | 1,44 (0,13) | 0,44 (0,07) |

Die Behandlung mit Taurin (0,5 %) führt zu keinen signifikanten Änderungen der Expression von HGF, KGF oder IGF-BP3. Demgegenüber konnte für die Behandlung mit Coffein (0,025 %) die tendenzielle Aktivierung der KGF und HGF Expression und die signifikante Deaktivierung des IGF-BP3 Gens nachgewiesen werden.

Überraschenderweise führte die Kombination von Coffein und Taurin zu einer besonders signifikanten Steigerung der Aktivierung der HGF-Genexpression. Zusätzlich führte die Behandlung mit dem Gemisch zur Verringerung der Aktivität des Gens für IGFBP-3 in synergistischer Weise. Hinsichtlich der Aktivität des Gens für KGF konnte für das Gemisch eine tendenzielle Steigerung nachgewiesen werden.

Die hier erhobenen Gen-Profile deuten auf Haarwuchs stimulierende und den vorzeitigen Haarausfall vermindernde Effekte hin, wobei durch die Kombination von Taurin und Coffein besonders ausgeprägte Effekte erreicht werden.

### Beispiel C: Nachweis der Freisetzung von Wachstumsfaktoren

Die beiden Wachstumsfaktoren Hepatocyte Growth Factor (HGF) und Keratinocyte Growth Factor (KGF) sind wichtige Regulatoren des Haarzyklus. Bei potentiell wachstumsfördemden und den vorzeitigen Haarverlust vermindernden Substanzen kann von einer Steigerung eines der ausgeschütteten Faktoren ausgegangen werden. Die Ausschüttung von HGF sowie KGF kann mit Hilfe von kommerziell erhältlichen ELISA-Kits quantifiziert werden.

Dazu wurden rekonstruierte dermale Papillen jeweils in Quadruplikaten über 24 h mit Taurin oder Coffein oder dem Gemisch dieser Substanzen gemäß Tabelle 2 inkubiert und die Konzentration der Wachstumsfaktoren im Medium in beschriebener Weise quantifiziert. Die Ergebnisse finden sich in Tabelle 5 wieder.

**Tabelle 5: KGF- und HGF-Freisetzung in [%] nach Behandlung rekonstruierter dermaler Papillen im Vergleich zu unbehandelten Kontrollen.**

| | **KGF-Freisetzung** | **HGF-Freisetzung** |
|---|---|---|
| | Mittelwert | Mittelwert |
| **Kontrolle** | 100 (9,0) | 100 (52,4) |
| **Gemisch** | 132 (17,1) | 286 (62,9) |
| **Taurin** | 110(11,5) | 152 (29,9) |
| **Coffein** | 119 (9,7) | 170 (35,8) |

Die Behandlung mit Coffein (0,025%) oder Taurin (0,5 %) führt nach Behandlung der rekonstruierten dermalen Papille zu einer leicht erhöhten Freisetzung von KGF um 10 bzw. 19 %. Bei Behandlung mit dem Gemisch zeigte sich eine Steigerung der KGF Ausschüttung um 32 % und damit eine deutlich ausgeprägtere Steigerung als theoretisch zu erwarten.

Bezüglich der HGF-Ausschüttung konnte für Coffein und Taurin eine Steigerung um 70 % bw. 52 % nachgewiesen werden. Unter Annahme additiver Effekte führt die Inkubation der dermalen Papillenzellen mit einer Kombination aus Taurin (0,5 %) und Coffein (0,025 %) zu einer Steigerung der HGF-Freisetzung um 122 %. Überraschenderweise wurde für dieses Gemisch allerdings eine Steigerung der HGF-Ausschüttung um 186 % nachgewiesen.

Diese Ergebnisse auf Proteinebene sind die konsequente Folge der Genexpressionsdaten aus Beispiel B. Im Vergleich zur Einzelsubstanzbehandlung führt die vermehrte Aktivierung der Gene für HGF und KGF auch zu einer gesteigerten Freisetzung von HGF und KGF.

Beide Wachstumsfaktoren sind wichtige Botenstoffe des Haarzyklus. Ausgehend von der aktuellen wissenschaftlichen Literatur kann an Hand dieser in vitro Daten von einer potenzierten, synergistischen Wirkung in vivo hinsichtlich Haarwuchs und Verminderung des vorzeitigen Haarausfalls durch Kombination von Coffein und Taurin ausgegangen werden.

## Patentansprüche

1. Kosmetisches Mittel, enthaltend in einem kosmetisch akzeptablen Träger, jeweils bezogen auf das gesamte Mittel
a) 0,001 bis 0,25 Gew.-% Coffein und
b) 0.01 bis 1 Gew.-% 2-Aminoethansulfonsäure.

2. Mittel nach Anspruch 1, **dadurch gekennzeichnet, dass** es weiterhin mindestens ein Silikonöl und/oder ein Silikongum enthält.

3. Mittel nach Anspruch 2, **dadurch gekennzeichnet, dass** das Silikonöl und/oder Silikongum ausgewählt ist aus der Gruppe, umfassend cyclische und lineare Polydialkylsiloxane, deren alkoxylierte und/oder aminierte Derivate, Dihydroxypolydimethylsiloxane und Polyphenylalkyl-siloxane

4. Mittel nach einem der Ansprüche 1 bis 3, **dadurch gekennzeichnet, dass** das Mittel zusätzliche Bestandteile enthält, ausgewählt aus der Gruppe, umfassend Proteinhydrolysate, filmbildende Substanzen, weitere haarwuchsstimulierende Wirkstoffe, nichtionogene Tenside, anionische Tenside, zwitterionische Tenside, ampholytische Tenside, nichtionische Polymere, Verdickungsmittel, Parfümöle, Farbstoffe, Lichtschutzmittel, Antioxidantien, Antischuppenwirkstoffe, Treibmittel, Reduktionsmittel.

5. Mittel nach einem der Ansprüche 1 bis 4, **dadurch gekennzeichnet, dass** es weiterhin mindestens ein kationisches Tensid ausgewählt aus quartären Ammoniumverbindungen, Esterquats und Amidoaminen enthält.

6. Mittel nach einem der Ansprüche 1 bis 5, **dadurch gekennzeichnet, dass** es zusätzlich bezogen auf das gesamte Mittel 0,01 bis 20 Gew.-% mindestens eines filmbildenden und/oder festigenden Polymers enthält.

7. Mittel nach Anspruch 6, **dadurch gekennzeichnet, dass** es sich um ein Stylinggel, ein Pumphaarspray, ein Aerosolhaarspray, einen Pumphaarschaum oder ein Aerosolhaarschaum handelt.

8. Mittel nach einem der Ansprüche 1 bis 7, zur Anwendung in einem Verfahren zur Vitalisierung von Haaren, Reaktivierung der Haarwurzel, Aktivierung von Haarfollikeln, Förderung oder Verstärkung des Haarwachstums, zur Haarkonditionierung, zur Verminderung von Haarausfall und/oder zur Behandlung von Haarausfall, **dadurch gekennzeichnet, dass** man das Mittel auf die Haare, Kopfhaut, behaarte Haut bzw. auf Hautstellen, wo der Haarwuchs seit längerem eingestellt ist, wo jedoch ein Haarwuchs gewünscht wird, aufbringt.

## Claims

1. A cosmetic agent containing, in a cosmetically acceptable carrier, based in each case on the total agent:
a) 0.001 to 0.25 wt% caffeine and
b) 0.01 to 1 wt% 2-aminoethanesulfonic acid.

2. The agent according to Claim 1, **characterised in that** it furthermore contains at least one silicone oil and/or one silicone gum.

3. The agent according to Claim 2, **characterised in that** the silicone oil and/or silicone gum is selected from the group encompassing cyclic and linear polydialkylsiloxanes, alkoxylated and/or aminated derivatives thereof, dihydroxypolydimethylsiloxanes and polyphenylalkylsiloxanes.

4. The agent according to one of Claims 1 to 3, **characterised in that** the agent contains additional constituents selected from the group encompassing protein hydrolysates, film-forming substances, further hair-growth-stimulating active substances, nonionogenic surfactants, anionic surfactants, zwitterionic surfactants, ampholytic surfactants, nonionic polymers, thickening agents, perfume oils, dyes, light protection agents, antioxidants, anti-dandruff active substances, propellants, reducing agents.

5. The agent according to one of Claims 1 to 4, **characterised in that** it further contains at least one cationic surfactant selected from quaternary ammonium compounds, esterquats and amidoamines.

6. The agent according to one of Claims 1 to 5, **characterised in that** it additionally contains, based on the total agent, 0.01 to 20 wt% of at least one film-forming and/or setting polymer.

7. The agent according to Claim 6, **characterised in that** it is a styling gel, a pump hair spray, an aerosol hair spray, a pump hair foam or an aerosol hair foam.

8. The agent according to one of Claims 1 to 7, for utilization in a method for vitalizing hair, reactivating the hair roots, activating hair follicles, promoting or intensifying hair growth, for hair conditioning, to minimize hair loss and/or for the treatment of hair loss, wherein the agent is applied onto the hair, scalp, hair-bearing skin resp. onto skin areas where hair growth has stopped for some time but where hair growth is desired.

## Revendications

1. Agent cosmétique contenant, dans un support acceptable du point de vue cosmétique, chaque fois rapportés à l'agent dans sa totalité,
a) de 0,001 à 0,25 % en poids de caféine ;
b) de 0,01 à 1 % en poids de l'acide 2-aminoéthanesulfonique.

2. Agent selon la revendication 1, **caractérisé en ce qu'**il contient en outre au moins une huile de silicone et/ou une gomme de silicone.

3. Agent selon la revendication 2, **caractérisé en ce que** l'huile de silicone et/ou la gomme de silicone sont choisies parmi le groupe comprenant des polydialkylsiloxanes cycliques et linéaires, leurs dérivés alcoxylés et/ou aminés, des dihydroxypolydiméthylsiloxanes et des polyphénylalkylsiloxanes.

4. Agent selon l'une quelconque des revendications 1 à 3, **caractérisé en ce que** l'agent contient des constituants supplémentaires, choisis parmi le groupe comprenant des hydrolysats de protéines, des substances filmogènes, d'autres substances actives qui stimulent la pousse des cheveux, des agents tensioactifs non ionogènes, des agents tensioactifs anioniques, des agents tensioactifs zwitterioniques, des agents tensioactifs ampholytiques, des polymères non-ioniques, des épaississants, des essences de parfums, des colorants, des agents de protection contre la lumière, des antioxydants, des substances actives antipelliculaires, des agents propulseurs, des agents de réduction.

5. Agent selon l'une quelconque des revendications 1 à 4, **caractérisé en ce qu'**il contient en outre au moins un agent tensioactif cationique choisi parmi des composés d'ammonium quaternaires, des esterquats et des amidoamines.

6. Agent selon l'une quelconque des revendications 1 à 5, **caractérisé en ce qu'**il contient en outre, rapportés à l'agent dans sa totalité, de 0,01 à 20 % en poids d'au moins un polymère filmogène et/ou renforçateur.

7. Agent selon la revendication 6, **caractérisé en ce qu'**il s'agit d'un gel coiffant, d'un spray capillaire à pomper, d'un spray capillaire à aérosol, d'une mousse capillaire à pomper ou d'une mousse capillaire à aérosol.

8. Agent selon l'une quelconque des revendications 1 à 7, pour son application dans un procédé pour la revitalisation des cheveux, à la réactivation des racines des cheveux, l'activation des follicules pileux, le développement ou le renforcement de la pousse des cheveux, pour la facilitation du démêlage des cheveux, pour la réduction de la chute des cheveux et/ou pour le traitement de la chute des cheveux, **caractérisé en ce qu'**on applique un agent sur les cheveux, sur le cuir chevelu, sur de la peau recouverte de poils, respectivement à des endroits de la peau auxquels la pousse des cheveux a cessé depuis un certain temps, mais où une pousse des cheveux est néanmoins souhaitée.
